# EUROPEAN PATENT SPECIFICATION

(11) **EP 1 303 304 B1**
(45) Date of publication and mention of the grant of the patent: **09.07.2008**
(21) Application number: 01954229.9
(22) Date of filing: 13.07.2001
(51) Int. Cl.: A61K 45/06, A61K 33/00, A61P 9/10, A61P 3/10, A61K 31/34, A61K 31/21

(54) **PHARMACEUTICAL COMBINATIONS FOR TREATMENT AND PREVENTION OF DIABETES MELLITUS**
PHARMAZEUTISCHE KOMBINATIONSPRÄPARATE ZUR BEHANDLUNG VON DIABETES MELLITUS
COMBINAISONS PHARMACEUTIQUES POUR TRAITER ET PREVENIR DIABETES MELLITUS

(30) Priority: 14.07.2000 HU 0002628
(43) Date of publication of application: 23.04.2003
(73) Proprietor: Kéri Pharma Generics Kft., Bartha Boldizsár u. 7 4032 Debrecen (HU)
(72) Inventor: SZILVASSY, Zoltán, H-4032 Debrecen (HU); TOSAKI, Arpád, H-4032 Debrecen (HU); NEMETH, József, H-7643 Pécs (HU); KOVACS, Péter, H-4032 Debrecen (HU); PANKUCSI, Csaba, H-4032 Debrecen (HU); HERNADI, Ferenc, H-4032 Debrecen (HU); FERNINANDY, Péter, H-6701 Szeged (HU)
(74) Representative: Flaccus, Rolf-Dieter
(86) International application number: PCT/HU2001/000079
(87) International publication number: WO 2002/005795

(56) References cited:
- EP-A- 0 845 265
- WO-A-96/08966
- WO-A-98/43621
- US-A- 5 698 589
- KHAN S.A. ET AL: "Role of calcium-activated K+ channels in vasodilation induced by nitroglycerine, acetylcholine and nitric oxide." JOURNAL OF PHARMACOLOGY AND EXPERIMENTAL THERAPEUTICS, (1993) 267/3 (1327-1335). , XP008007178

## Description

The object of this invention is a pharmaceutical combination for treatment and prevention of diabetes mellitus, including all sorts and periods of diabetes mellitus, thus including the pre-diabetic diseases comprising optionally more than one pharmaceutical composition, whereby at least one of the compositions comprises an effective dose of at least one enzymatic nitric oxide (NO) donor and optionally an effective dose of at least one antidiabetic active ingredient and optionally the usual pharmaceutically acceptable carriers and/or other auxiliaries.

The basis of the invention is the recognition of a new insulin-sensitizing effect and synergism using enzymatic NO donors for monotherapy or in combined therapy with a conventional antidiabetic active ingredient, mainly with a per os antidiabetic active ingredient. It was found that besides the known vascular effect enzymatic NO donors have a metabolic (hypoglycaemic/antihyperglycaemic) effect as well. Thus the present invention represents a fundamentally new antidiabetic therapeutic approach using an antianginal agent with metabolic effect.

Where not otherwise stated the following definitions and abbreviations are used further on:
Diabetes : all sorts and periods of diabetes mellitus, including all diseases associated with diabetes mellitus, and pre-diabetic diseases and their complications.
As diabetes-associated diseases, disturbances in gastric and intestinal motility, such as gastroparesis and problems of sphincter of ODDI are mentioned. Insulin-dependent (Type I.) diabetes mellitus: IDDM Non-insulin dependent (Type II.)diabetes mellitus: NIDDM Polycistic ovary syndrome (diabetic disease): PCOS Gestational diabetes syndrome (pre-diabetic disease): GDM nitroglycerin (enzymatic NO donor): NTG racemic isosorbide mononitrate, and/or its stereoizomers : (enzymatic NO donor): ISMN racemic isosorbide dinitrate and/or its stereoizomers: (enzymatic NO donor): ISDN 3-morpholinosydnonimine (non-enzymatic NO donor): SIN-1

It is known that IDDM results from a decreased insulin production by the pancreatic β-cells. NIDDM is known as a heterogeneous disease resulting from a dynamic interaction between defects in insulin secretion and insulin action.
Mechanisms responsible for the β-cell failure are not totally clarified, but may be related to the chronic demands placed on the β-cells by peripherial insulin resistance and/or the effect of hyperglycemia to impair β-cell function. The β-cell failure may also occur as an independent inherent defect in "pre-diabetic" individuals. NIDDM often develops from certain at risk populations, such as indviduals with polycistic ovary syndrome which is the most common endocrine disorder in women of reproductive age.

NO donor drugs are widely used in ischaemic heart disease and for the treatment of cardiac failure. Such medications can be used in a variety of formulations with different can be used in a variety of formulations with different routes of administration: parenteral (intravenous, intramuscular, subcutaneous) transdermal (patch, oinment), rectal or enteral (sublingual, buccal, per os in liquid or solid forms).
NO donors have two basic groups: non-enzymatic NO donors, and enzymatic NO donors. Non-enzymatic NO donors release NO spontaneuosly by chemical degradation, while enzymatic NO donors require an enzymatic process.
Non-enzymatic NO donors include sydnonimine-derivatives (SIN-1), sodium nitropusside, S-nitroso-N-acetyl-D,L-penicillamine, sodium nitrite.
Enzymatic NO donors include NTG, ISMN, ISDN, erythrityl tetranitrate, pentaerythritol tetranitrate, methyl-propyl-propanediol-dinitrate, propatylnitrate, trolnitrate, tenitramine, nicorandile. Nitroglycerin is a prototype of the enzymatic NO donors.
Recently, the non-enzymatic NO donor SIN-1 has been reported to inhibit insulin release in isolated pancreatic islets (Am. J. Physiol 1996;271;C1098-C1102). Insulin sensitivity was further reported to be increased through stimulation of NO production in the liver (patent application No PCT/US/99/23098) using non-enzymatic NO donors such as SIN-1, sodium nitrite, sodium nitropusside, and S-nitroso-N-acetyl-D,L-penicillamine.

Using enzymatic NO donors is a basic recognition of our invention, while non-enzymatic NO donors, such as SIN-1 are not suitable for treatment of diabetes mellitus. This is summarized as follows:
It is known that enzymatic NO donors do not release NO in coronary vessels with a diameter smaller than 100 µm. Thus although dilating supepicardial arteries including stenotic segments and collateral vessels in the coronary vasculature they do not dilate coronary microvessels i.e. resistance coronary vessels. This selective effect renders enzymatic NO donors 'safe coronary vasodilators' due to the minimum or no risk of the 'coronary steal' phenomenon characteristic for other pure vasodilators such as slow Ca²⁺ channel blockers or phosphodiesterase inhibitors. Non-enzymatic NO donors however (due to spontaneous NO release) are known to dilate coronary conductance (>100 µm) and resistance (<100 µm) vessels, as well.
Beyond vascular effect, enzymatic NO donors are known to elicit several favourable biological actions, such as inhibition of platelet aggregation, inhibition Ca²⁺ entry into cardiac myocytes, inhibition of catecholamine release from cardiac adrenergic nerve terminals, and other actions in the central nervous system and immune system as well.
Non-enzymatic NO-donors have several unfavourable effects as summarized below:
According to recent publications, NO produced by non-enzymatic NO donors play an important role in the destruction of the pancreatic β-cells thereby leading to IDDM. Therefore, any therapeutic use of the non-enzymatic NO donors to enhance insulin-sensitivity in NIDDM is clinically contraindicated. It means, that the said compounds worsen diabetes mellitus. Furthermore, the mortality of the so-called 'deadly quartet' (NIDDM, obesity, dislypidemia, and hypertension) is mainly (80%) due to ischemic heart disease and/or cardiac failure. During chemical degradation SIN-1 is known to produce super-oxide anion (O₂⁻) and NO, and the chemical reaction of these two radicals gives peroxinitrite (ONOO⁻), the toxic properties of which are well known (Exp: Toxicol Pathol 1999;51:517-21).

Also cytotoxic effects of non-enzymatic NO donors were reported.
In a clinical investigation (Metabolism 2000;49:313-318), the hypothesis was tested that sodium nitroprusside would increase insulin-mediated glucose uptake in humans. In the control group insulin was infused using the euglycemic clamp protocol. It was found that systemic infusion of sodium nitroprusside did not increase insulin-mediated glucose disposal neither in young nor in old subjects.

It is known that NIDDM can be treated initially using monotherapy with known oral antidiabetic agents (such as sulphonylureas, biguanides, α-glucosidase inhibitors, benzoic acid derivatives, thiazolidinediones, α2-receptor antagonists) but will eventually require the combination of said compounds, and in most patients, an additional insulin therapy will be needed. Long-term control of blood glucose levels in IDDM and NIDDM will decrease the incidence and prolong the time until progression but will not inhibit complications such as diabetic retinopathy, nephropathy, and neuropathy. (J. Natl.Med. Assoc. 1991, 91, 389-395; Ann Intern Med. 1999, 131, 281-303).

The recognition of our invention thus includes:
a.) monotherapy with an enzymatic NO donor results in a hypoglycaemic/antihyperglycaemic effect in humans and other mammals, and
b.) the combination of an enzymatic NO donor, with an antidiabetic active ingredient, particularly a per os agent, results in an increase of the hypoglycaemic/antihyperglycaemic effect of the antidiabetic active ingredient while providing protection against myocardial ischaemia, a commonly occurring complication of NIDDM, and further that fect is reduced due to the insulin sensitizing effect of the enzymatic NO donor.

A first object of the present invention concerns the use of at least one enzymatic nitric oxide (NO) donor organic nitrate for the manufacture of an insulin-sensitizing medicament for: treatment of diabetes mellitus; prevention of diabetes mellitus; treatment of pre-diabetes and pre-diabetic diseases; prevention of pre-diabetic diseases; treatment of a diabetes-associated disease selected from the group consisting of: disturbances in gastric and intestinal motility, particularly gastroparesis; problems affecting the sphincter of ODDI;
wherein said medicament contains an insulin-sensitizing effective dose of said at least one enzymatic NO donor organic nitrate.

A further object of the present invention relates to the use of at least one enzymatic nitric oxide (NO) donor organic nitrate for the manufacture of an insulin-sensitizing medicament for the treatment of: disturbances of gall bladder motility, gall stone diseases in diabetes; wherein said medicament contains an insulin-sensitizing effective dose of said at least one enzymatic nitric oxide (NO) donor organic nitrate in combination with an antidiabetic sulphonylurea derivative.

A further object of the present invention concerns an insulin-sensitizing medicament comprising an insulin-sensitizing effective dose of at least one enzymatic nitric oxide (NO) donor organic nitrate and at least one anti-diabetic active ingredient selected from the group consisting of thiazolidinedion, biguanide derivatives, α-glucosidase inhibitor, α2-adrenergic antagonist, sulphonamides, preferably a sulphonyl urea, troglitazone, pioglitazone, rosiglitazone, meglitinide analogues, acetohexamide, carbutamide, chlorpropamide, glibenclamide, glibornuride, glibutamide, gliclazide, glipizide, glimeperide, gliquidone, glisentide, glisolamide, glisoxepide, glybuzole, glyclopyramide, glycyclamide, glymidine free acid and its salts, metahexamide, tolazamide, tolbutamide, metformine, phenformine, buformine, idazoxane, acarbose, miglitol and voglibose.

Further embodiments of the above-defined invention are described in the dependent patent claims.

It is evident from the above the combinations according to the invention include three embodiments of the invention : the use of a formulation (composition) containing a NO-donor, a formulation containing both a NO-donor and an antidiabetic together and more than one formulation (composition) where the NO-donor and the antidiabetic appear in separated formulations.

Another aspect of the invention concerns the use of at least one enzymatic nitric oxide (NO) donor organic nitrate for the manufacture of an insulin-sensitizing medicament for the treatment and prevention of pre-diabetic diseases, such as polycistic ovary syndrome (PCOS), and of gestational diabetes syndrome (GDM).

The combination or composition may comprise an organic nitrate compound as enzymatic NO donor, and insulin or a per os antidiabetic active ingredient, preferably thiazolidinedion, biguanide derivative, α-glucosidase-inhibitor, α2-adrenergic-antagonist and/or a sulphonamide, preferably a sulphonylurea as the antidiabetic active ingredient.

According to the preferred embodiment the enzymatic NO donor is nitroglycerin (NTG), racemic isosorbide mononitrate, and/or its stereoizomers (ISMN), racemic isosorbide dinitrate and/or its stereoizomers (ISDN), erythrityl tetranitrate, pentaerytritol-tetranitrate, methylpropyl-propanediol-dinitrate, propatyl nitrate, trolnitrate, tenitramine and/or nicorandile,
and the antidiabetic active ingredient is insulin, troglitazone, pioglitazone, rosiglitazone, meglitinide analogues, acetohexamide, carbutamide, chlorpropamide, glibenclamide, glibornuride, glibutamide, gliclazide, glipizide, glimepiride, gliquidone, glisentide, glisolamide, glisoxepide, glybuzole, glyclopyramide, glycyclamide, glymidine free acid and its salts, metahexamide, tolazamide, tolbutamide, metformine, phenformine, buformine, idazoxane, acarbose, miglitol, and/or voglibose.

An important embodiment of the invention is a combination or composition comprising as the enzymatic NO donor nitroglycerin, racemic isosorbide mononitrate and/or its stereoizomers, racemic isosorbide dinitrate and/or its stereoizomers, and as the antidiabetic active ingredient insulin, troglitazone, pioglitazone, rosiglitazone, glibenclamide, metformine and/or idazoxane.

The compositions according to the invention may be formulated for direct medical use for parenteral (intravenous, intramuscular, subcutaneous), transdermal (patch or oinment), per os liquid and solid (tablet, spray, liquid), rectal, nasal, sublingual, buccal administration for controlled (sustained) or usual release.

Another important aspect of our invention is, that we have found, that the effective doses related to the new insulin-sensitizing effect are considerably lower than the usual doses related to the known effect of most active substances (see data of the following preferred embodiments). Our results have shown for the first time that in addition to favourable effects on the heart and vasculature, nitroglycerin, isosorbide-5-mononitrate, and all of the other enzymatic NO donors at a dose lower than used for the treatment of stable angina pectoris (see Table 1) produces metabolic effects that influence insulin sensitivity in healthy and insulin resistant patients and mammals.

The higher dose is only necessary, when the patient has also to be treated against the " classical" disease, the ischaemic heart disease.

Preferred embodiments of our invention are formulations comprising the following daily or per hour effective doses for NO-treatment alone:

| | |
|---|---|
| NTG sustained-release, p.os. | 0,5-31.2 mg |
| NTG transdermal oinment | 0.2-0.8 mg/hour |
| NTG transdermal patch | 0.2-0.8 mg/hour |
| ISDN tablet, capsule | 0,3-135 mg |
| ISDN sustained-release, p.os. | 0,2-160 mg |
| ISDN transdermal | 3-180 mg |
| ISMN tablet, capsule | 1-40 mg |
| ISMN sustained-release, p.os | 2-240 mg |
| ISMN transdermal | 40-300 mg |

A preferred combination or composition comprises the following combinations of two active ingredients using the following daily or per hour effective doses
a.) as enzymatic NO donor:

| | |
|---|---|
| NTG retard per os | 0.26-31.2 mg |
| NTG transdermal | 0.02-0.8 mg/hour |
| NTG transd. oinment | 0.02-0.8 mg/hour |
| ISDN per os | 0,1-135 mg |
| ISDN retard per os | 0,2-160 mg |
| ISDN transdernal | 3-180 mg |
| ISMN per os | 0,1-120 mg |
| ISMN retard per os | 0,2-240 mg |
| ISMN transdermal | 3-300 mg |

b.) as per os antidiabetic active ingredient:

| | |
|---|---|
| glibenclamide, per os | 0,75-14 mg |
| metformin, per os | 50-3000 mg |
| glyburide | 0.1-100 mg |
| idazoxan, per os | 20-600 mg |
| troglitazon | 20-600 mg |
| pioglitazon | 5- 50 mg |
| rosiglitazon | 5- 50 mg |
| insulin, i.v. | 4-500 NE/ml. |

For the purpose of our invention more than two active ingredients are applicable. The following variations are preferred combinations using effective doses as exemplified above:
enzymatic NO donor, sulphonylurea, and/or biguanide derivative; or
enzymatic NO donor, sulphonylurea, and/or thiazolidinedione derivative; or
enzymatic NO donor, sulphonylurea derivative and/or insulin; or
enzymatic NO donor, biguanide, and/or tiadiazolidindion derivative;
or
enzymatic NO donor, biguanide derivative and/or insulin; or enzymatic NO donor, thiazolidinedione derivative and/or insulin; or
enzymatic NO donor, sulphonylurea, biguanide, and/or thiazolidinedione derivative; or
enzymatic NO donor, sulphonylurea, biguanide,
thiazolidinedione derivative and/or insulin.

In the following we summerize some of the main benefits of our invention:
Nitroglycerin a prototype of enzymatic NO donors enhance insulin sensitivity in humans, and thus combination of sulphonylureas with an enzymatic NO donor yielded an amalgamation of benefit in controlling NIDDM metabolic disorder epecially in patients at risk of ischeamic heart disease.

Another aspect of the benefit from a sulphonylurea - enzymatic NO donor combination derives from the prevalence of gastrointestinal motility disorders in diabetes. These alterations are considered to result from the other major complication of diabetes i.e. peripheral neuropathy. As a result, disturbances occur in gastric and intestinal motility (diabetic gastroparesis) as well as in gall-bladder and sphincter of Oddi motility. The latter is of crucial importance in the development of gall-stone disease in diabetes. Moreover, K_{ATP} activation is an important mechanism in nitrergic relaxation of the sphincter of Oddi, thus, sulphonylurea derivatives may at least in part block physiological sphincter of Oddi relaxation function even in the absence of diabetes. Therefore when a sulphonylurea derivative is combined with as enzymatic NO donor, beyond producing a synergistic effect on glucose metabolism, the combination bears preservation of gastrointestinal sphincter function with special regards to the sphincter of Oddi. Finally, as the antidiabetic dose of a sulphonylurea in the presence of enzymatic NO donors is lower than that used in monotherapy, the risk of hypoglycaemia will also be lower.

Metformin one of the biuanides treat obese NIDDM patients. However, besides inducing lactate acidosis of low incidence, this drug was contraindicated in cardiac and respiratory insufficiency. Due to the insulin sensitizing effect of enzymatic NO donors, a potentiating synergism between enzymatic NO donors and metformin on blood glucose lowering effect allow the antidiabetic dose of metformin to decrease, moreover, the vascular effects and direct myocardial protection induced by anzymatic NO donors would make metformin therapy safer in NIDDM patients at risk of myocardial disease.

The major disadvantage of α-glucosidase inhibitors was gastrointestinal intolerance due to both osmotic effects and bacterial fermentation of undigested carbohydrates. These effects, however, exhibited dose dependence.

Since enzymatic NO donors further reduce insulin release with a potentiating effect on the hypoglyceamic performance of insulin, an enzymatic NO donor - acerbose combination would yield a reduction in the antihyperglyceamic dose of acarbose, therefore reducing its gastrointestinal side effects.

The major disadvantage of the use of thiazolidinediones derives from their dose-dependent toxic effects produced aneamia and liver damage. Combining thiazolidinediones with enzymatic NO donors reduced the antidiabetic dose of thiazolidinediones resulting in a decrease in their potential to produce toxic effects. Moreover, the protective effect of the two drugs against myocardial ischaemia further decreases the incidence of ischaemic heart disease in NIDDM patients.

In diabetic state, the insulin sensitivity enhancing efficacy of enzymatic NO donors overcome their inhibitory effect on insulin release in favour of a hypoglycaemic action at modest insulin release. In addition, enzymatic NO donors counteract the hypertensive effect of α₂-receptor antagonists besides conferring protection on the ischaemic heart.

Since enzymatic NO donors enhance insulin sensitivity, the combination of insulin with enzymatic NO donors necessitates lower and less frequently applied insulin doses when insulin is the therapeutic possibility.

### Explanation of the Figures:

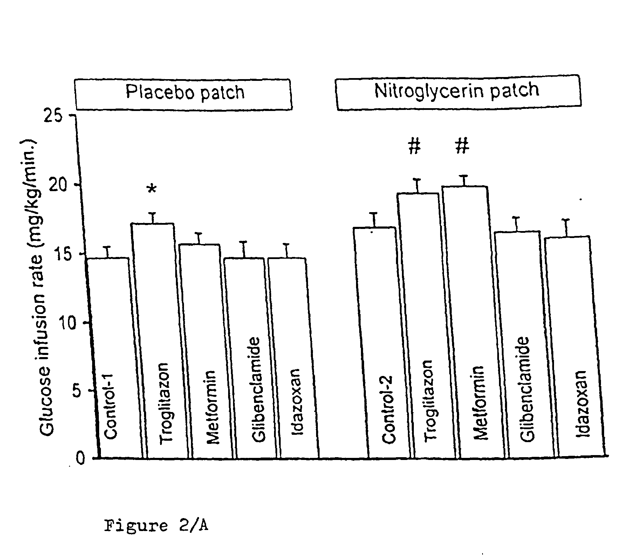

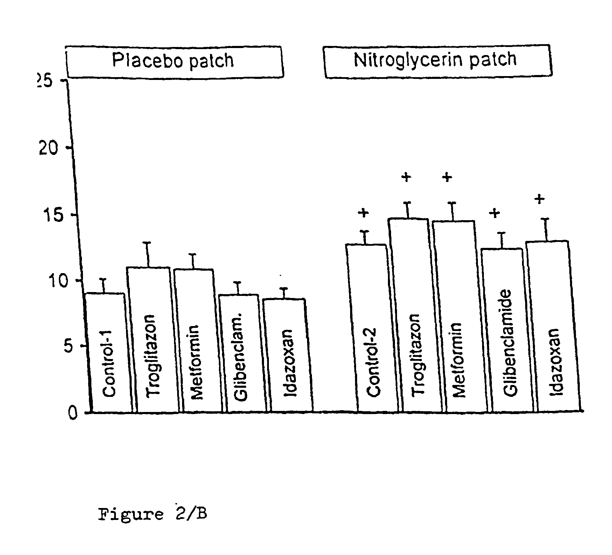

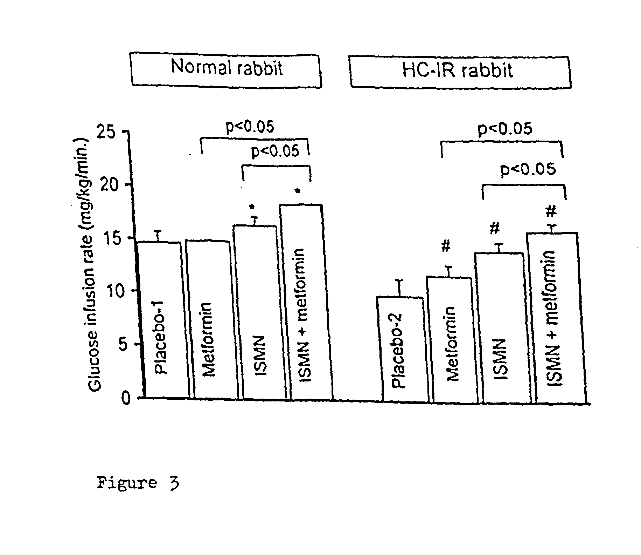

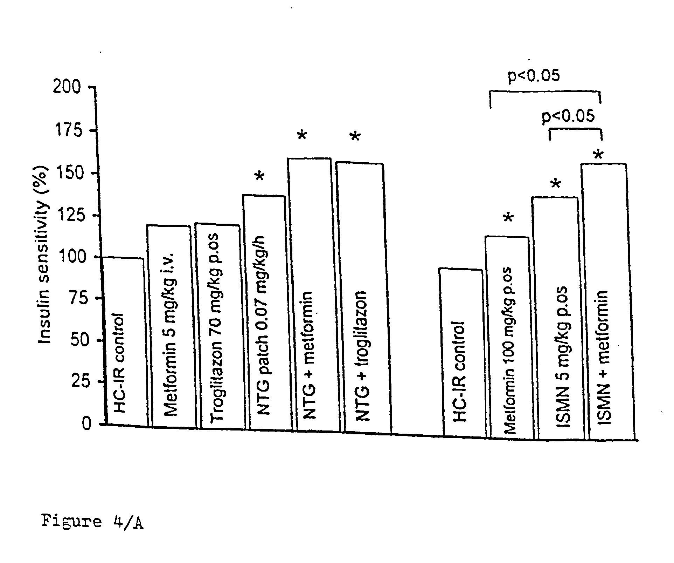

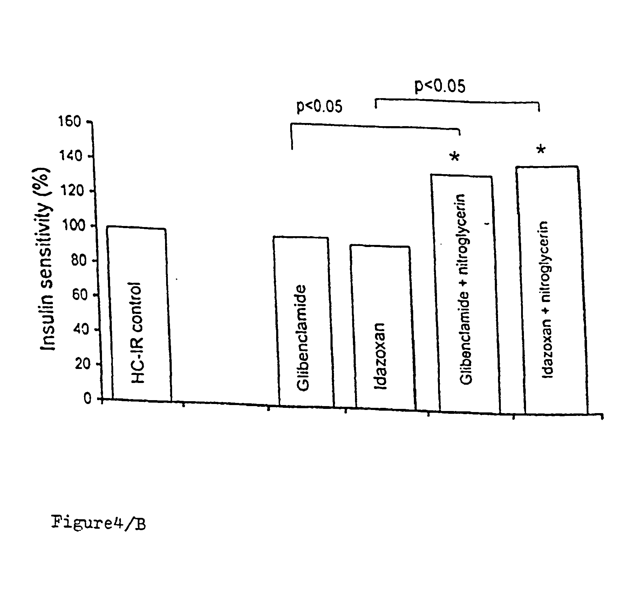

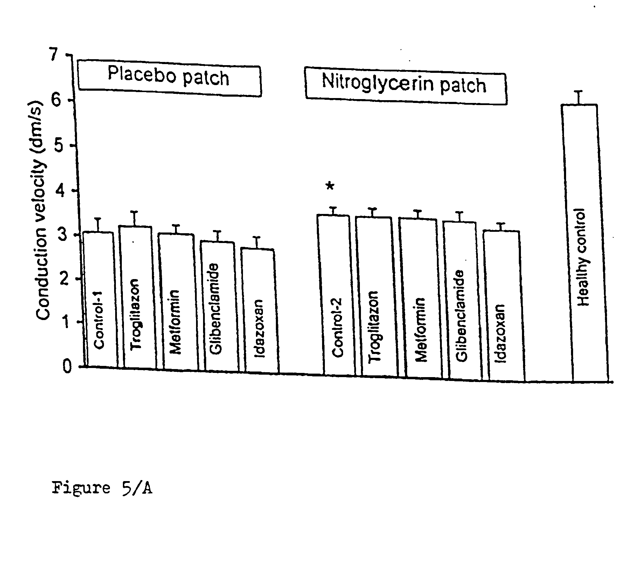

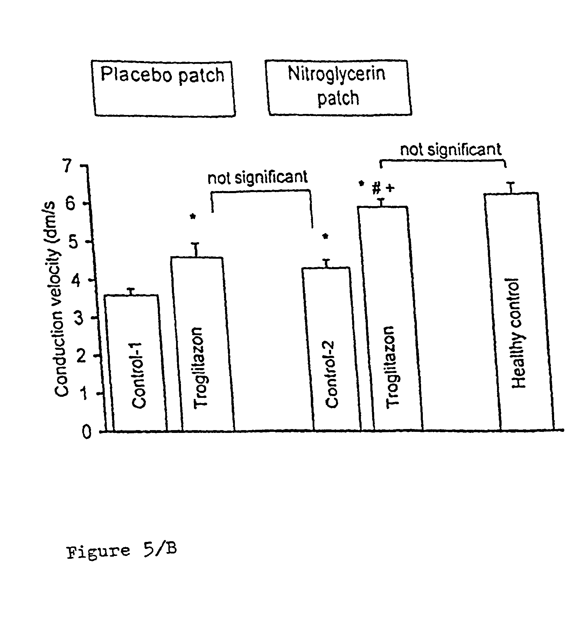

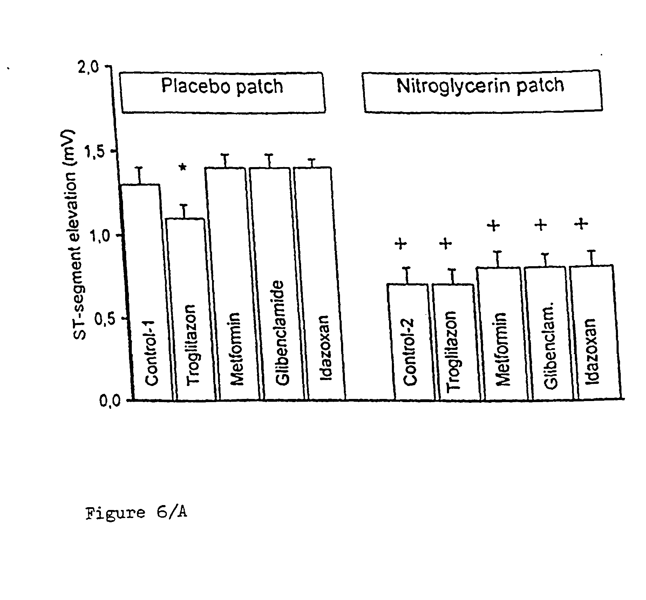

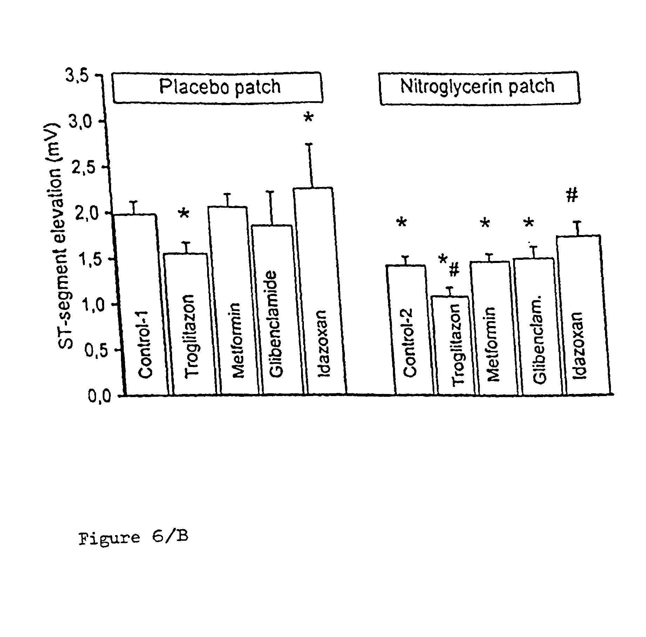

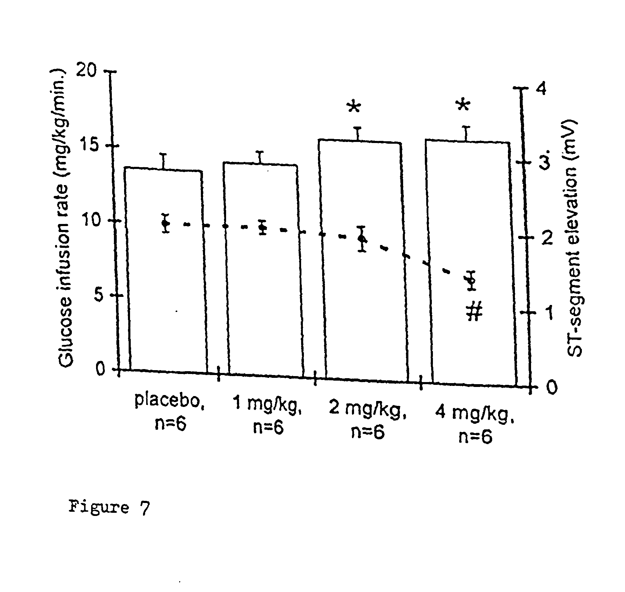

Figure 1. Effect of nitroglycerin patch (0.4 mg/hour) on oral glucose tolerance test in healthy volunteers. The data are means+/-SD, * placebo vs. active patch at p<0.05
   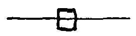 Placebo patch, insulin, n=20
   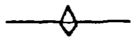 Active patch, insulin, n=20
   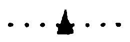 Placebo patch, glucose, n=20
   ····X··· Active patch, glucose, n=20
Figure 2/A Interaction between different treatments on insulin sensitivity in normal conscious rabbits. The data are means+/-SD.
   Control-1, n=6; Control-2, n=7; Nitroglycerin patch 0.07 mg/kg/h; Troglitazon 70 mg/kg p.os, n=6; Metformin 5 mg/kg i.v., n=6; Glibenclamide 1 mg/kg i.v., n=6; Idazoxan 2 mg/kg
   i.v., n=6
   *: significant vs. control-1, p<0.05
   #: significant vs. control-2, p<0.05
Figure 2/B Interaction between different treatments on insulin sensitivity in hypercholesterolaemic, insulin resistant conscious rabbits. The data are means+/-SD
   Control-1, n=6; Control-2, n=6; Nitroglycerin patch 0.07 mg/kg/h; Troglitazon 70 mg/kg p.os, n=6; Metformin 5 mg/kg i.v., n=6; Glibenclamide 1 mg/kg i.v., n=6; Idazoxan 2 mg/kg
   i.v., n=6
   +: significant vs. placebo patch, p<0.05
Figure 3 Interaction between ISMN and metformin on insulin sensitivity in normal and hypercholesterolaemic, insulin resistant-conscious rabbits. The data are means+/-SD
   Placebo-1, n=6; Placebo-2, n=6; Metformin 100 mg/kg p.os, n=6
   ISMN: isosorbide-5-mononitrate 5 mg/kg p.os n=6;
   HC-IR: hypercholesterolaemic, insulin resistant
   *: significant vs. placebo-1, p<0.05
   #: significant vs. placebo-2, p<0.05
Figure 4/A Synergism between enzymatic NO donors and insulin sensitizers in hypercholesterolaemic, insulin resistant conscious rabbits. The data are expressed as percent of means. HC-IR: hypercholesterolaemic, insulin resistant
   NTG: nitroglycerin; ISMN: isosorbide-5-mononitrate
   *: significant vs. HC-IR control, p<0.05
Figure 4/B Synergism between nitroglycerin and non insulin sensitizig antihyperglycaemic compounds in hypercholesterolaemic, insulin resistant conscious rabbits. The data are expressed as percent of means.
   HC-IR control, n=6; HC-IR: hypercholesterolaemic, insulin resistant; Nitroglycerin patch 0.07 mg/kg/h; Glibenclamide 1 mg/kg i.v., n=6; Idazoxan 2 mg/kg i.v., n=6; Combinations, n=6
   *: significant vs. HC-IR control, p<0.05
Figure 5/A Effect of different treatments on nerve conduction velocity in femoral "C" fibers in streptozotocin diabetic rabbits. The data are means+/-SD.
   Control-1, n=6; Control-2, n=6; Nitroglycerin patch 0.07 mg/kg/h; Troglitazon 70 mg/kg p.os, n=6; Metformin 5 mg/kg i.v., n=6; Glibenclamide 1 mg/kg i.v., n=6; Idazoxan 2 mg/kg
   i.v., n=6
   *: significant vs. control-1, p<0.05
Figure 5/B Effect of nitroglycerin and nitroglycerin-troglitazon combination on nerve conduction velocity in femoral "C" fibers in hypercholesterolaemic, insulin resistant rabbits. The data are means+/-SD.
   Contrcl-1, n=6; Control-2, n=6; Nitroglycerin patch 0.07 mg/kg/h; Troglitazon 70 mg/kg p.os, n=6
   *: significant vs. control-1, p<0.05
   #: significant vs. control-2, p<0.05
   +: significant vs. placebo patch, p<0.05
Figure 6/A Interaction between different treatments on ventricular pacing induced ischaemia in normal conscious rabbits. The data are means+/-SD
   Control-1, n=6
   Control-2, n=6; Nitroglycerin patch 0.07 mg/kg/h; Troglitazon
   70 mg/kg p.os, n=6; Metformin 5 mg/kg i.v., n=6; Glibenclamide
   1 mg/kg i.v., n=6; Idazoxan 2 mg/kg i.v., n=6
   *: significant vs. control-1, p<0.05
   +: significant vs. placebo patch, p<0.05
Figure 6/B Interaction between different treatments on ventricular pacing-induced ischaemia in hypercholesterolaemic, insulin resistant rabbits. The data are means+/-SD
   Control-1, n=6; Control-2, n=6; Nitroglycerin patch 0.07 mg/kg/h; Troglitazon 70 mg/kg p.os, n=6; Metformin 5 mg/kg i.v., n=6; Glibenclamide 1 mg/kg i.v., n=6; Idazoxan 2 mg/kg
   i.v., n=6
   *: significant vs. control-1, p<0.05
   #: significant vs. control-2, p<0.05
Figure 7 Treshold doses of per os controlled-release isosorbide-5-mononitrate (ISMN) on insulin sensitivity and myocardial ischaemia in normal conscious rabbits. The data are means+/-SD. * placebo vs. treatment on insulin sensitivity and # placebo vs. treatment on ST-elevation, p<0.05.
   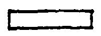 Glucose infusion rate (insulin sensitivity)
   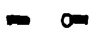 ST-elevation (ventricular pacing-induced ischaemia)

The following examples illustrate various aspects of the invention without the aim of limitation.

### Examples

### Method 1.

Effect of nitroglycerin on glucose-stimulated insulin release in humans.
Twenty persons were studied. None of them had a history of hypertension, diabetes mellitus, or smoking.

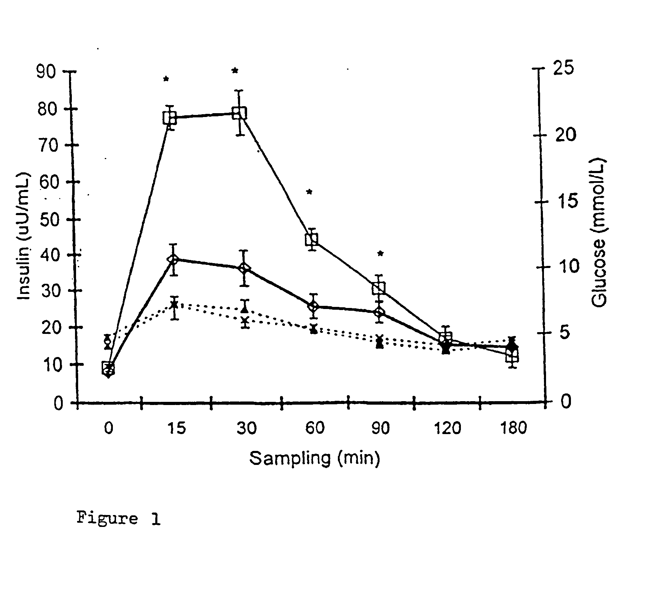

Between 2 oral glucose, tolerance tests (OGTT) the volunteers were randomized for receiving either active NITRODERM TTS 10 (releasing approx. 0.4 mg hour⁻¹ nitroglycerin) or placebo patches. The study was carried out and evaluated in a double blind fashion. The patients received the glucose solution at 8 a.m. Venous blood samples were taken during fasting (immediately before glucose) and at 15, 30, 60, 90, 120 and 180 minutes after the glucose load. The samples evaluated for plasma glucose level (mmol 1⁻¹) and immunoreactive insulin responses ( 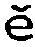 U ml⁻¹). Plasma glucose was determined by means of an autoanalyzer, using the glucose oxidease method. Immunoreactive insulin levels were assessed by radioimmunoassay method using antihuman antibody. During the study, two ECG leads (II and V₁ or V₆) and arterial blood pressure were continuously monitored.
Nitroglycerin significantly decreased the increase in insulin in response tooral glucose load as compared to corresponding values in the placebo patch group. There were no differences in plasma glucose levels in the two groups (Fig. 1 and Table I).

### Method 2.

### Animals and surgery:

The experiments are carried out with adult, male New Zealand white rabbits, weighing 3.0-3.5 kg, housed as described in Am. J. Physiol 1994, Heart Circ. Physiol, 35 : H2033-H2041 and J. Moll Cell. Cardiol. 1997, 29: 1977-1983.
Cardiac electrophysiology and haemodynamics:
The right intracavitary electrogram, the chest-lead ECG, the left ventricular pressure curve and MABP are continuously recorded as above.
Global myocardial ischaemia induced by ventricular overdrive pacing:
The induction of global myocardial ischaemia was based on a method described as above.
Experimental hyperlipidaemia and insulin resistance in rabbits:
Adult male New Zealand white rabbits are fed laboratory chow enriched with 1.5% cholesterol (atherosclerotic group) over a period of eight to twelve weeks. According to our experiences, exposure to cholesterol-enriched diet over this period results in a nearly twentyfold increase in serum total cholesterol level, a decrease in insulin-stimulated glucose uptake with aortic lesion surface area of 55 % and an approx. fifty percent loss of endothelium-dependent relaxation of rings from thoracic aortae in rabbits.
Nerve conduction velocity studies :
These series of experiments were carried out to verify/exclude sensory neuropathy. Left saphenous nerve conduction velocity was determined as described in Eur J. Pharmacol 1999, 386:83-88.
Determination of insulin sensitivity/insulin resistance :
To estimate insulin sensitivity and/or insulin resistance, hyperinsulinaemic (100 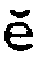 U/ml) euglycaemic (5.5 mM/l) glucose clamping was used. The glucose clamping values (M) were established as values of glucose infusion rates expressed in mg/kg/min to maintain blood glucose level at 5.5 mM/l.

### Results

### 1. Interaction between nitroglycerin and glibenclamide, metformin, troglitazon or idazoxan on insulin sensitivity in normal conscious rabbits:

As shown in Fig. 2/A and Table II, the M values of hyperinsulinaemic and euglycaemic glucose clamping significantly increased in the presence of troglitazon (70 mg/kg/day p. os over 3 days). Either metformin (5 mg/kg/day i.v. over 3 days) or glibenclamide (1 mg/kg i.v. 10 min prior to glucose infusion) or idazoxan (2 mg/kg i.v.) was without effect.

The M values are also significantly increased in the presence of 0.07 mg/kg/min transdermal nitroglycerin combined with troglitazon or metformin (Fig. 2/A, Table II).

### 2. Interaction between nitroglycerin and glibenclamide, metformin, troglitazon or idazoxan on insulin sensitivity in hypercholesterolaemic, insulin resistant conscious rabbits:

In the presence of nitroglycerin (0,07 mg/kg/h) M values of hyperinsulinaemic and euglycaemic glucose clamping increased as shown in Fig. 2/B (control-2, Table II). In the presence of nitroglycerin and troglitazon (70 mg/kg/day p.os over 3 days), metformin (5 mg/kg/day i.v. over 3 days), glibenclamide (1 mg/kg i.v. 10 min prior to glucose infusion) or idazoxan (2 mg/kg i.v.) the M values are also significantly increased compared to the placebo patch group (Fig. 2/B, Table II).

### 3. Interaction between isosorbide-5-mononitrate and metformin, on insulin sensitivity in normal conscious rabbits:

After treatment with isosorbide-5-mononitrate (ISMN) (1 mg/kg/day, Olicard 40, Solvay Pharma, Hannover, Germany) M values of hyperinsulinaemic and euglycaemic glucose clamping increased as compared to the control (Fig. 3, placebo-1; Table III) value. Metformin (100 mg/kg/day p.os over 3 days) was without any effect, but in the presence of ISMN , metformin elicited a significant increase in the M values (Fig. 3, Table III).

### 4. Interaction between isosorbide-5-mononitrate and metformin, on insulin sensitivity in hypercholesterolaemic, insulin resistant conscious rabbits:

After treatment with isosorbide-5-mononitrate (ISMN) (5 mg/kg/day, Olicard 40, Solvay Pharma, Hannover, Germany) M values of hyperinsulinaemic and euglycaemic glucose clamping significantly increased as compared to the control (Fig. 3, placebo-2) value. Metformin (100 mg/kg/day p.os over 3 days) alone and the combination of ISMN and metformin also elicited a significant increase in the M values (Fig. 3, Table III).
1.

### 5. Synergism between enzymatic NO donors and insulin sensitizer compounds on insulin sensitivity in hypercholesterolaemic, insulin resistant conscious rabbits:

The enzymatic NO donors (nitroglycerin, ISMN) combined with known insulin sensitizer compounds (metformin, troglitazon) produced synergism (increased effectivity) on insulin sensitivity, as shown in Fig. 4/B and Table IV.

### 6. Synergism between enzymatic NO donors and non insulin sensitising antihyperglycaemic compounds in hypercholesterolaemic, insulin resistant conscious rabbits:

Nitroglycerin combined with antihyperglycaemic compounds that are without insulin sensitising effect (glibenclamide, idazoxan) produced synergism (increased effectivity) on glycaemic control (Fig. 4/B, Table IV).

Nitroglycerin and glibeclamide combination produced synergism not only on metabolic effects, but on anti-ischaemic effects (Fig. 6/B, Table VT), as well.

### 7. Interaction between nitroglycerin and glibenclamide, metformin, troglitazon or idazoxan on femoral nerve conduction velocity in anaesthetized rabbits with streptozotocin diabetes:

Streptozotocin (40 mg/kg i.v.) produced a decrease in nerve conduction velocity in femoral "A" and "C" fibres, respectively, as determined 8 weeks after streptozotocin injection. Nitroglycerin (12-h patch on vs 12-h patch off periods over three days) significantly improved nerve conduction in "C" fibres (Fig. 5/A, Table V) with a marginal amelioration of "A" fibre conduction (data not shown). Neither troglitazon,nor glibenclamide, idazoxan or metformin produced any effect on nerve conduction velocity in the streptozotocin-diabetes model. The combination of any of these drugs with nitroglycerin yielded an improvement of conduction velocity in streptozotocin-diabetic aminals similar to that seen with nitroglycerin alone.

### 8. Synergism between enzymatic NO donors and insulin sensitizer compounds on femoral nerve conduction velocity in anaesthetized rabbits with hyperlipidaemia-induced insulin resistance:

An eight-week period of atherogenic diet resulted in a decrease in nerve conduction velocity in "C" fibres (Fig. 5/B.; control-1; Table V) . This was attenuated by nitroglycerin and troglitazon (Fig. 5/B, control-2; Table V).
When these drugs were applied together, the "C" fibre nerve conduction velocity values increased further, and did not differ significantly from those measured in healthy rabbits in the absence of any drugs (Fig. 5/B, Table V).

### 9. Interaction between nitroglycerin and glibenclamide, metformin, troglitazon or idazoxan on pacing-induced myocardial ischaemia in normal conscious rabbits:

Troglitazon and nitroglycerin significantly decreased intra cavitary ST-segment elevation (Fig. 6/A, Table VI), but met formin and glibenclamide were without effect.
When nitroglycerin was combined with troglitazon, glibencla mide, metformin or idazoxan, each combination produced an anti-ischaemic effect similar to that produced by nitroglyc erin alone (Fig 6/A, Table VI).

### 10. Interaction between nitroglycerin and glibenclamide, metformin, troglitazon or idazoxan on pacing-induced myocardial ischaemia in hypercholesterolaemic, insulin resistant conscious rabbits:

Nitroglycerin significantly decreased intracavitary ST-segment elevation (Fig. 6/B; control-2; Table VI). Troglitazon also induced an anti-ischaemic effect although of lower amplitude than that produced by nitroglycerin. Metformin and glibenclamide were without effect. Idazoxan aggravated ischaemic changes produced by VOP (Fig. 6/B, Table VI), moreover, VOP induced ventricular extrasystoles in the insulin resistant animals.
When nitroglycerin was combined with troglitazon the anti-ischaemic effect exceeded that induced by nitroglycerin alone (Fig 6/B, Table VI). Nitroglycerin + metformin, nitroglycerin + glibenclamide produced an anti-ischaemic effect approx. of the same magnitude as that seen with nitroglycerin alone.

### 11. Threshold doses of per os extended-release isosorbide-5-mononitrate on insulin sensitivity and ventricular pacing-induced myocardial ischaemia in normal conscious rabbits:

The effect of 1, 2, and 4 mg/kg per os ISMN were tested, to compare the metabolic (insulin sensitising) and anti-ischaemic (changes in ST-segment elevation) dose range of ISMN. 1 mg/kg was without any effect, but 2 mg/kg produced significant metabolic effect with a statistically insignificant anti-ischaemic effect. 4 mg/kg and higher doses of ISMN had metabolic and anti-ischaemic effects, as well (Fig. 7, Table VII). The threshold dose of the insulin sensitising effect was 2mg/kg, 50% of the threshold dose of the anti-ishaemic effect.
The usual human anti-anginal (anti-ischaemic) doses of controlled release ISMN preparations are 30 to 240 mg/day. The threshold anti-anginal dose is 30 mg/day.
Human dose: 15 mg retard ISMN + 250 mg retard metfromin Evaluation of Experimental Results:
Our results have shown for the first time that in addition to favourable effects on the heart and vasculature, nitroglycerin, ISMN, and all of the other enzymatic NO donors at a dose identical or even lower to that used for the treatment of stable angina pectoris (see Table 1) produces metabolic effects, that influence insulin sensitivity in healthy and insulin resistant patients and mammals.
The enzymatic NO-donors applied at an anti-ischaemic dosage range (see the corresp. Table) produces additional metabolic effects, that influence post-prandial haemodynamic adjustments of potential risk in patients and mammals with coronary artery disease.

The combinations of the known antihyperglycaemic compounds with enzymatic NO donors produce synergism on insulin sensitivity.

**Table 1**

| Doses of the Enzymatic NO-Donors Used for the Treatment of Stable Angina Pectoris | | | | |
|---|---|---|---|---|
| | drug | form | mg | daily administration |
| 1. | NTG | sublingual tablet | 0,3-0,6 | 1-3 times or 2-5 min. before activity |
| 2. | NTG | sublingual spray | 0,4-0,8 | 1-3 times or 2-5 min. before activity |
| 3. | NTG | buccal tablet | 1-3 | 3 times or 2-5 min. before activity |
| 4. | NTG | sustained release, oral | 2,6-10,4 | 2-3 times |
| 5. | NTG | transdermal ointment, 2% | 1-10 cm 7,5-30 mg | 3-4 times |
| 6. | NTG | transdermal patch | 0,2-0,8 mg/bour | once |
| 7. | ISDN | oral spray | 1,25-3,75 | 1-3 times or 2-5 min. before activity |
| 8. | ISDN | sublingual tablet | 2,5-10 | 5-10 min, before activity |
| 9. | ISDN | tablet, capsule | 10-45 | 3 times |
| 10. | ISDN | sustained release, oral | 20-80 | 1-2 times |
| 11. | ISDN | transdermal | 30-90 | 1-2 times |
| 12. | ISMN | tablet, capsule | 10-20 | twice |
| 13. | ISMN | sustained release, oral | 30-240 | once |

**Table 2. Effect of transdermal nitroglycerin on oral glucose tolerance test (OGTT) in healthy volunteers.**

| | Placebo patch | Active patch | | |
|---|---|---|---|---|
| Sampling | Glucose, mmol l⁻¹ | Insulin, µU ml⁻¹ | Glucose, mmol l⁻¹ | Insulin, µU ml⁻¹ |
| 0(conlrol) | 4.8±0.2 | 9.1±0.9 | 4.2±0.3 | 8.2±0.9 |
| 15 min | 7.4±0.5' | 77.7±3.3⁺ | 7.3±1.1⁺ | 38.9±4.4"⁺ |
| 30 min | 6.9±0.7' | 78.8±6.1⁺ | 6.1±0.6⁺ | 36.3±5.0'⁺ |
| 60 min | 5.3±0.4⁺ | 44.3±3.1⁺ | 5.5±0.4 | 25.6±3.3'⁺ |
| 90 min | 4.4±0.2 | 30.6±3.7⁺ | 4.7±0.7 | 24.1±2.9'⁺ |
| 120 min | 3.8±0.4 | 16.8±3.3⁺ | 4.2±0.3 | 15.2±2.4' |
| 180 min | 4.1±0.4 | 12.1±3.1 | 4.5±0.5 | 14.4±2.6 |

| | | | | |
|---|---|---|---|---|
| The data are means±S.D. obtained with 20 patients. +: different from '0' values at p<0.05; * placebo vs active patch at p<0.05. | | | | |

| **Table I Effect of transdermal nitroglycerin on oral glucose tolerance test in human volunteers** | | | | |
|---|---|---|---|---|
| | **Placebo patch** | | **Active patch** | |
| **Sampling (min)** | **Glucose(mmoL⁻¹)** | **Insulin (µUmL⁻¹)** | **Glucose(mmoL⁻¹)** | **Insulin (µUmL⁻¹)** |
| 0 (control) | 4.8±0.2 | 9.1±0.9 | 4.2±0.3 | 8.2±0.9 |
| 15 | 7.4±0.5 | 77.7±3.3 | 7.3±1.1 | 38.9±4.4 |
| 30 | 6.9±0.7 | 78.8±6.1 | 6.1±0.6 | 36.3±5.0 |
| 60 | 5.3±0.4 | 44.3±3.1 | 5.5±0.4 | 25.6±3.3 |
| 90 | 4.4±0.2 | 30.6±3.7 | 4.7±0.7 | 24.1±2.9 |
| 120 | 3.8±0.4 | 16.8±3.3 | 4.2±0.3 | 15.2±2.4 |
| 180 | 4.1±0.4 | 12.1±3.1 | 4.5±0.5 | 14.4±2.6 |
| The data are mean±SD obtained from 20 patients. | | | | |

| **Table II Interaction between different treatments on insulin sensitivity in rabbits** | | | | |
|---|---|---|---|---|
| | **Glucose infusion rate (mg/kg/min.)** | | | |
| | **Normal rabbit** | | **Hypercholesterolaemic, insulin resistant rabbit** | |
| | **Placebo patch** | **Active patch (nitroglycerin 0.07 mg/kg/h)** | **Placebo patch** | **Active patch (nitroglycerin 0.07 mg/kg/h)** |
| Control | 14.667±0.8165 n=6 | 16.857±1.0690 n=7 | 9.000±1.0954 n=6 | 12.667±1.0328 n=6 |
| Troglitazon, n=6 (70 mg/kg p.os) | 17.167±0.7528 | 19.333±1.0328 | 11.000±1.8974 | 14.667±1.2111 |
| Metformin, n=6 (5 mg/kg i.v.) | 15.667±0.8165 | 19.833±0.7528 | 10.833±1.1690 | 14.5001±1.3784 |
| Glibenclamide, n=6 (1 mg/kg i.v.) | 14.667±1.2111 | 16.500±1.0488 | 8.833±0.9832 | 12.333±1.2111 |
| Idazoxan, n=6 (2 mg/kg i.v.) | 14.667±1.0328 | 16.000±1.2649 | 8.500±0.8367 | 12.833±1.7224 |
| The data are mean±SD. | | | | |

| **Table III Interaction between ISMN and metformin on insulin sensitivity in rabbits** | | |
|---|---|---|
| | **Glucose infusion rate (mg/kg/min.)** | |
| **Treatment** | **Normal rabbit** | **Hypercholesterolaemic, insulin resistant rabbit** |
| Control, n=6 | 14.60±1.0300 | 9.70±1.6000 |
| Metformin, n=6 (100 mg/kg p.os) | 14.80±0.0800 | 11.70±1.0300 |
| ISMN controlled release, (5 mg/kg p.os) | 16.30±0.8200 | 14.00±0.8900 |
| ISMN controlled release (5 mg/kg p.os) + metformin (100 mg/kg P.os), n=6 | 19.30±0.0820 | 15.90±0.7500 |
| The data are mean±SD. | | |

| **Table IV Synergism between enzymatic NO donors and known anti-diabetics in hyperchotesterolaemic, insulin resistant rabbits** | |
|---|---|
| **Treatment** | **Insulin sensitivity (%)** |
| Control | 100.0 |
| Metformin (5 mg/kg i.v.) | 120.4 |
| Troglitazon (70 mg/kg p.os) | 122.2 |
| Nitroglycerin patch (0.07 mg/kg/h) | 140.7 |
| Nitroglycerin patch (0.07 mg/kg/h) + metformin (5 mg/kg i.v.) | 163.0 |
| Nitroglycerin patch (0.07 mg/kg/h) + troglitazon (70 mg/kg p.os) | 161.1 |
| Metformin (100 mg/kg p.os) | 120.6 |
| ISMN controlled release (5 mg/kg p.os) | 144.4 |
| ISMN controlled release (5 mg/kg p.os) + Metformin (100 mg/kg p.os) | 163.9 |

| **Table V Interaction between different treatments on nerve conduction velocity in femoral** **"C" fibers in rabbits** | | | | |
|---|---|---|---|---|
| | **Nerve conduction velocity (dm/s))** | | | |
| **Healthy untreated rabbit** | 6.2±0.3 | | | |
| | **Streptozotocin diabetic rabbit** | | **Hypercholesterolaemic, insulin resistant rabbit** | |
| **Treatment** | **Placebo patch** | **Active patch** | **Placebo patch** | **Active patch** |
| Control, n=6 | 3.1±0.3 | 3.6±0.2 | 3.6±0.2 | 4.3±0.4 |
| Troglitazon, n=6 | 3.2±0.3 | 3.6±0.2 | - | - |
| (70 mg/kg p.os) | | | | |
| Metformin, n=6 | 3.1±0.2 | 3.6±0.2 | - | - |
| (5 mg/kg i v.) | | | | |
| Glibenclamide, n=6 | 2.9±0.24 | 3.6±0.2 | - | - |
| (1 mg/kg i.v.) | | | | |
| Idazoxan, n=6 | 2.8±0.26 | 3.4±0.2 | - | - |
| (2 mg/kg i.v.) | | | | |
| | | | | |
| Troglitazon, n=6 (70 mg/kg p.os) | - | - | 4.6±0.4 | 5.9±0.2 |
| The data are mean±SD. | | | | |

| **Table VI Interaction between dilfferent treatments on ventricular pacing-induced ischaemia in rabbits** | | | | |
|---|---|---|---|---|
| | **ST-segment elevation (mV)** | | | |
| **Treatment** | **Normal rabbit** | | **Hypercholesterolaemic, insulin resistant rabbit** | |
| | **Placebo patch** | **Active patch (nitroglycerin 0.07 mg/kg/h)** | **Placebo patch** | **Active patch (nitroglycerin 0.07 mg/kg/h)** |
| Control, n=6 | 1.3±0.10 | 0.7±0.10 | 2.0±0.13 | 1.4±0.10 |
| Troglitazon, n=6 | 1.1±0.08 | 0.7±0.09 | 1.6±0.12 | 1.08±0.10 |
| (70 mg/kg p.os) | | | | |
| Metformin, n=6 | 1.4±0.08 | 0.8±0.10 | 2.1±0.13 | 1.5±0.08 |
| (5 mg/kg i v.) | | | | |
| Glibenclamide, n=6 | 1.4±0.08 | 0.8±0.08 | 1.9±0.40 | 1.5=0.13 |
| (1 mg/kg i.v.) | | | | |
| Idazoxan, n=6 | 1.4±0.05 | 0.8±0.09 | 2.3±0.50 | 1.8±0.16 |
| (2 mg/kg i.v.) | | | | |
| The data are mean±SD. | | | | |

| **Table VII Treshold doses of per os controlled release ISMN on insulin sensitivity and myocardial ischaemia in normal rabbits** | | |
|---|---|---|
| **Treatment** | **Glucose infusion rate (mg/kg/min.)** | **ST-segment elevation (mV)** |
| Control, n=6 | 13.6±1.03 | 2.0±0.12 |
| | | |
| 1 mg/kg ISMN, n=6 | 14.2±0.8 | 2.0±0.09 |
| 2 mg/kg ISMN, n=6 | 16.0±0.9 | 1.9±0.16 |
| 4 mg/kg ISMN, n=6 | 16.3±1.0 | 1.4±0.12 |
| | | |
| The data are mean±SD | | |

## Claims

1. The use of at least one enzymatic nitric oxide (NO) donor organic nitrate for the manufacture of an insulin-sensitizing medicament for:
- treatment of diabetes mellitus;
- prevention of diabetes mellitus;
- treatment of pre-diabetes and pre-diabetic diseases;
- prevention of pre-diabetic diseases;
- treatment of a diabetes-associated disease selected from the group consisting of:
disturbances in gastric and intestinal motility, particularly gastroparesis; problems affecting the sphincter of ODDI;
wherein said medicament contains an insulin-sensitizing effective dose of said at least one enzymatic NO donor organic nitrate.

2. The use of at least one enzymatic NO donor as claimed in claim 1, **characterized in that** said pre-diabetic diseases are selected from the group of polycystic ovary syndrome and gestational diabetes syndrome.

3. The use of an enzymatic nitric oxide (NO) donor organic nitrate as claimed in claim 1, wherein said medicament is a pharmaceutical composition containing said NO donor(s), and which optionally comprises pharmaceutically acceptable carriers and/or other auxiliaries, said composition being preferably formulated as a composition for oral liquid, oral solid, nasal, or sublingual administration, and most preferably in tablet form.

4. The use of an enzymatic nitric oxide (NO) donor organic nitrate as claimed in any one of claims 1 to 3, wherein said medicament further comprises an effective dose of at least one antidiabetic active ingredient, preferably an effective dose of at least one oral antidiabetic active ingredient.

5. The use of an enzymatic nitric oxide (NO) donor organic nitrate as claimed in claim 4, wherein said medicament is a pharmaceutical composition containing both said NO donor(s) and said antidiabetic ingredient(s), and optionally comprises pharmaceutically acceptable carriers and/or other auxiliaries.

6. The use of an enzymatic nitric oxide (NO) donor organic nitrate as claimed in claim 4, wherein said medicament comprises a combination of at least two separate pharmaceutical compositions, and wherein one of said at least two compositions comprises an effective dose of said at least one NO donor, and at least one further composition of said combination comprises an effective dose of said at least one antidiabetic ingredient, and said composition(s) optionally comprise(s) pharmaceutically acceptable carriers and/or other auxiliaries.

7. The use of an enzymatic nitric oxide (NO) donor organic nitrate as claimed in any one of claims 1 to 6, wherein said NO donor(s) is/are selected from the group consisting of nitroglycerin (NTG), racemic isosorbide mononitrate (ISMN) and/or its stereoisomers, racemic isosorbide dinitrate (ISDN) and/or its stereoisomers, erythrityl tetranitrate, pentaerythritol tetranitrate, methylpropyl-propanediol dinitrate, propatyl nitrate, trolnitrate, tenitramine and nicorandile.

8. The use of an enzymatic nitric oxide (NO) donor organic nitrate as claimed in any one of claims 4 to 7, wherein said antidiabetic ingredient(s) is/are selected from the group consisting of insulin, thiazolidinedion, biguanide derivatives, α-glucosidase inhibitor, α2-adrenergic antagonist, sulphonamides, preferably a sulphonyl urea, troglitazone, pioglitazone, rosiglitazone, meglitinide analogues, acetohexamide, carbutamide, chlorpropamide, glibenclamide, glibornuride, glibutamide, gliclazide, glipizide, glimeperide, gliquidone, glisentide, glisolamide, glisoxepide, glybuzole, glyclopyramide, glycyclamide, glymidine free acid and its salts, metahexamide, tolazamide, tolbutamide, metformine, phenformine, buformine, idazoxane, acarbose, miglitol and voglibose.

9. The use of an enzymatic nitric oxide (NO) donor organic nitrate as claimed in any one of claims 4 to 8, wherein said NO donor(s) is/are selected from the group consisting of nitroglycerin, racemic isosorbide mononitrate and/or its stereoisomers, racemic isosorbide dinitrate and/or its stereoisomers, and wherein said antidiabetic ingredient is selected from the group consisting of insulin, troglitazone, pioglitazone, rosiglitazone, glibenclamide, metformine and idazoxane.

10. The use of an enzymatic nitric oxide (NO) donor organic nitrate as claimed in any one of claims 3 to 9, wherein said composition(s) is/are formulated for parenteral, preferably intravenous, intramuscular, subcutaneous or transdermal, or for oral, nasal, sublingual or buccal administration, wherein said oral formulations comprise liquid preparations, preferably liquids and sprays, and solid preparations, preferably tablets.

11. The use of an enzymatic nitric oxide (NO) donor organic nitrate as claimed in claim 10, wherein said composition(s) is/are formulated as a sustained-release formulation, preferably as a sustained-release medicament for oral application or a transdermal patch.

12. The use of an enzymatic nitric oxide (NO) donor organic nitrate as claimed in claim 3, wherein said composition contains the following daily doses, or per hour doses, of nitroglycerin (NTG), isosorbide mononitrate (ISMN) or isosorbide dinitrate (ISDN):
| | |
|---|---|
| NTG sustained-release, per os: | 0.5-31.2 mg; |
| NTG transdermal patch: | 0.2-0.8 mg/h; |
| ISDN tablet or capsule: | 0.3-135 mg; |
| ISDN sustained release, per os: | 0.2-160 mg; |
| ISDN transdermal: | 3-180 mg; |
| ISMN tablet or capsule: | 1-40 mg; |
| ISMN sustained-release, per os: | 2-240 mg; |
| ISMN transdermal: | 40-300 mg. |

13. The use of an enzymatic nitric oxide (NO) donor organic nitrate as claimed in claim 3, wherein said composition contains the following daily doses of nitroglycerin (NTG), isosorbide mononitrate (ISMN) or isosorbide dinitrate (ISDN):
| | |
|---|---|
| NTG sustained-release, per os: | 0.5-5.2 mg; |
| ISDN tablet or capsule: | 0.3-3 mg; |
| ISDN sustained release, per os: | 0.2-2 mg; |
| ISMN tablet or capsule: | 1-20 mg; |
| ISMN sustained-release, per os: | 2-30 mg; |
| ISMN transdermal: | 40-300 mg. |

14. The use of an enzymatic nitric oxide (NO) donor organic nitrate as claimed in any one of claims 4 to 11, wherein said medicament is intended for combined therapy treatment, and said medicament is a composition as defined in claim 5 or comprises a combination of compositions as defined in claim 6, and said composition(s) contain(s) the following doses of NO donor (a) and antidiabetic ingredient (b), respectively:
a) as enzymatic NO donor:
| | |
|---|---|
| NTG retard, per os | 0.26-31.2 mg; |
| ISDN, per os | 0.1-135 mg; |
| ISDN retard, per os | 0.2-160 mg; |
| ISDN transdermal | 3-180 mg; |
| ISMN per os | 0.1-120 mg; |
| ISMN retard per os | 0.2-240 mg; |
b) and, as antidiabetic active ingredient:
| | |
|---|---|
| glibenclamide, per os | 0.75-14 mg; |
| metformin, per os | 50-3000 mg; |
| glyburide, per os | 0.1-100 mg; |
| idazoxan, per os | 20-600 mg; |
| troglitazon, per os | 20-600 mg; |
| pioglitazon, per os | 5-50 mg; |
| rosiglitazon, per os | 5-50 mg; |
| insulin, i.v. | 4-500 NE/ml. |

15. The use of an enzymatic nitric oxide (NO) donor organic nitrate as claimed in any one of claims 4 to 11, wherein said medicament said comprises one of the following combinations of two or more active substances:
- enzymatic NO donor, sulphonylurea and biguanide derivative; or
- enzymatic NO donor, sulphonylurea and thiazolidinedione derivative; or
- enzymatic NO donor, sulphonylurea and insulin; or
- enzymatic NO donor, biguanide derivative, and thiazolidinedione derivative; or
- enzymatic NO donor, biguanide derivative, and insulin; or
- enzymatic NO donor, thiazolidinedione derivative and/or insulin; or
- enzymatic NO donor, sulphonylurea, biguanide and thiazolidinedione derivative; or
- enzymatic NO donor, sulphonylurea, biguanide, thiazolidinedione derivative and insulin.

16. An insulin-sensitizing medicament comprising an insulin-sensitizing effective dose of at least one enzymatic nitric oxide (NO) donor organic nitrate and at least one anti-diabetic active ingredient selected from the group consisting of thiazolidinedion, biguanide derivatives, α-glucosidase inhibitor, α2-adrenergic antagonist, sulphonamides, preferably a sulphonyl urea, troglitazone, pioglitazone, rosiglitazone, meglitinide analogues, acetohexamide, carbutamide, chlorpropamide, glibenclamide, glibornuride, glibutamide, gliclazide, glipizide, glimeperide, gliquidone, glisentide, glisolamide, glisoxepide, glybuzole, glyclopyramide, glycyclamide, glymidine free acid and its salts, metahexamide, tolazamide, tolbutamide, metformine, phenformine, buformine, idazoxane, acarbose, miglitol and voglibose.

17. A medicament as claimed in claim 16, **characterized in that** said medicament is a pharmaceutical composition containing both said NO donor(s) and said antidiabetic ingredient(s), and optionally comprises pharmaceutically acceptable carriers and/or other auxiliaries.

18. A medicament as claimed in claim 16, **characterized in that** said medicament comprises a combination of at least two separate pharmaceutical compositions, and wherein one of said at least two compositions comprises an effective dose of said at least one NO donor, and at least one further composition of said medicament comprises an effective dose of said at least one antidiabetic ingredient, and said composition(s) optionally comprise(s) pharmaceutically acceptable carriers and/or other auxiliaries.

19. A medicament as claimed in any one of claims 16 to 18, **characterized in that** said NO donor(s) is/are selected from the group consisting of nitroglycerin (NTG), racemic isosorbide mononitrate (ISMN) and/or its stereoisomers, racemic isosorbide dinitrate (ISDN) and/or its stereoisomers, erythrityl tetranitrate, pentaerythritol tetranitrate, methylpropyl-propanediol dinitrate, propatyl nitrate, trolnitrate, tenitramine and nicorandile.

20. A medicament as claimed in any one of claims 17 to 19, **characterized in that** said pharmaceutical composition(s) is/are formulated for parenteral, preferably intravenous, intramuscular, subcutaneous or transdermal, or for oral, nasal, sublingual or buccal administration, wherein said oral formulations comprise liquid preparations, preferably liquids and sprays, and solid preparations, preferably tablets.

21. A medicament as claimed in any one of claims 16 to 20, **characterized in that** it further contains insulin.

22. A medicament as claimed in any one of claims 16 to 21, **characterized in that** it comprises one of the following combinations of two or more active substances:
- enzymatic NO donor, sulphonylurea and biguanide derivative; or
- enzymatic NO donor, sulphonylurea and thiazolidinedione derivative; or
- enzymatic NO donor, sulphonylurea and insulin; or
- enzymatic NO donor, biguanide derivative, and thiazolidinedione derivative; or
- enzymatic NO donor, biguanide derivative, and insulin; or
- enzymatic NO donor, thiazolidinedione derivative and/or insulin; or
- enzymatic NO donor, sulphonylurea, biguanide and thiazolidinedione derivative; or
- enzymatic NO donor, sulphonylurea, biguanide, thiazolidinedione derivative and insulin.

## Patentansprüche

1. Verwendung wenigstens eines als enzymatischer Stickstoffoxid(NO)-Donator wirkenden organischen Nitrats für die Herstellung eines insulinsensibilisierenden Medikaments zur:
- Behandlung von Diabetes mellitus
- Prophylaxe von Diabetes mellitus
- Behandlung von Prädiabetes und prädiabetischen Erkrankungen
- Prophylaxe prädiabetischer Erkrankungen
- Behandlung einer diabetesassoziierten Erkrankung, die ausgewählt ist aus der Gruppe bestehend aus:
Störungen der gastrointestinalen Motilität, insbesondere Gastroparese, und den Sphinkter Oddi betreffende Probleme;
wobei das Medikament eine insulinsensibilisierend wirkende Dosis des wenigstens einen, als enzymatischer NO-Donator wirkenden organischen Nitrats enthält.

2. Verwendung wenigstens eines enzymatischen NO-Donators nach Anspruch 1, **dadurch gekennzeichnet, dass** die prädiabetischen Erkrankungen aus der Gruppe ausgewählt sind, die aus polyzystischem Ovarialsyndrom und Gestationsdiabetes-Syndrom besteht.

3. Verwendung eines als enzymatischer Stickstoffoxid(NO)-Donator wirkenden organischen Nitrats nach Anspruch 1, bei welcher das Medikament eine pharmazeutische Zusammensetzung ist, welche den bzw. die NO-Donator(en) enthält und wahlweise pharmazeutisch akzeptable Träger und/oder andere Hilfsstoffe umfasst, wobei die Zusammensetzung vorzugsweise als eine Zusammensetzung für die orale Verabreichung in flüssiger Form, die orale Verabreichung in fester Form, die nasale oder die sublinguale Verabreichung formuliert ist, besonders bevorzugt in Tablettenform.

4. Verwendung eines als enzymatischer Stickstoffoxid(NO)-Donator wirkenden organischen Nitrats nach einem der Ansprüche 1 bis 3, bei welcher das Medikament des Weiteren eine wirksame Dosis wenigstens eines antidiabetischen Wirkstoffs, vorzugsweise eine wirksame Dosis wenigstens eines oralen antidiabetischen Wirkstoffs, enthält.

5. Verwendung eines als enzymatischer Stickstoffoxid(NO)-Donator wirkenden organischen Nitrats nach Anspruch 4, bei welcher das Medikament eine pharmazeutische Zusammensetzung ist, die sowohl den bzw. die NO-Donator(en) als auch den bzw. die antidiabetischen Wirkstoff(e) enthält und wahlweise pharmazeutisch akzeptable Träger und/oder andere Hilfsstoffe umfasst.

6. Verwendung eines als enzymatischer Stickstoffoxid(NO)-Donator wirkenden organischen Nitrats nach Anspruch 4, bei welcher das Medikament eine Kombination von wenigstens zwei getrennten pharmazeutischen Zusammensetzungen umfasst, wobei eine der zumindest zwei Zusammensetzungen eine wirksame Dosis des wenigstens einen NO-Donators umfasst, wenigstens eine weitere Zusammensetzung der Kombination eine wirksame Dosis des wenigstens einen antidiabetischen Wirkstoffs umfasst und die Zusammensetzung(en) wahlweise pharmazeutisch akzeptable Träger und/oder andere Hilfsstoffe umfasst bzw. umfassen.

7. Verwendung eines als enzymatischer Stickstoffoxid(NO)-Donator wirkenden organischen Nitrats nach einem der Ansprüche 1 bis 6, bei welcher der bzw. die NO-Donator(en) aus der Gruppe ausgewählt ist/sind, die aus Nitroglycerin (NTG), racemischem Isosorbidmononitrat (ISMN) und/oder dessen Stereoisomeren, racemischem Isosorbiddinitrat (ISDN) und/oder dessen Stereoisomeren, Erythrityltetranitrat, Pentaerythritoltetranitrat, Methylpropylpropandioldinitrat, Propatylnitrat, Trolnitrat, Tenitramin und Nicorandil besteht.

8. Verwendung eines als enzymatischer Stickstoffoxid(NO)-Donator wirkenden organischen Nitrats nach einem der Ansprüche 4 bis 7, bei welcher der bzw. die antidiabetische(n) Wirkstoff (e) aus der Gruppe ausgewählt ist/sind, die aus Insulin, Thiazolidindion, Biguanid-Derivaten, α-Glucosidase-Inhibitor, α2-adrenergem Antagonist, Sulfonamiden, vorzugsweise einem Sulfonylharnstoff, Troglitazon, Pioglitazon, Rosiglitazon, Meglitinid-Analoga, Acetohexamid, Carbutamid, Chlorpropamid, Glibenclamid, Glibornurid, Glibutamid, Gliclazid, Glipizid, Glimeperid, Gliquidon, Glisentid, Glisolamid, Glisoxepid, Glybuzol, Glyclopyramid, Glycyclamid, Glymidin (freie Säure and ihre Salze), Metahexamid, Tolazamid, Tolbutamid, Metformin, Phenformin, Buformin, Idazoxan, Acarbose, Miglitol und Voglibose besteht.

9. Verwendung eines als enzymatischer Stickstoffoxid(NO)-Donator wirkenden organischen Nitrats nach einem der Ansprüche 4 bis 8, bei welcher der bzw. die NO-Donator(en) aus der Gruppe ausgewählt ist/sind, die aus Nitroglycerin, racemischem Isosorbidmononitrat und/oder dessen Stereoisomeren, racemischem Isosorbiddinitrat und/oder dessen Stereoisomeren besteht, und bei welcher der antidiabetische Wirkstoff aus der Gruppe ausgewählt ist, die aus Insulin, Troglitazon, Pioglitazon, Rosiglitazon, Glibenclamid, Metformin und Idazoxan besteht.

10. Verwendung eines als enzymatischer Stickstoffoxid(NO)-Donator wirkenden organischen Nitrats nach einem der Ansprüche 3 bis 9, bei welcher die Zusammensetzung(en) für die parenterale, vorzugsweise intravenöse, intramuskuläre, subkutane oder transdermale, oder für die orale, nasale, sublinguale oder bukkale Verabreichung formuliert ist/sind und die oralen Formulierungen flüssige Zubereitungen, vorzugsweise Flüssigkeiten und Sprays, und feste Zubereitungen, vorzugsweise Tabletten, umfassen.

11. Verwendung eines als enzymatischer Stickstoffoxid(NO)-Donator wirkenden organischen Nitrats nach Anspruch 10, bei welcher die Zusammensetzung(en) als eine Retard-Formulierung, vorzugsweise als ein Retard-Medikament für die orale Verabreichung oder ein transdermales Pflaster, formuliert ist/sind.

12. Verwendung eines als enzymatischer Stickstoffoxid(NO)-Donator wirkenden organischen Nitrats nach Anspruch 3, bei welcher die Zusammensetzung die nachfolgend genannten Tagesdosen oder stündlichen Dosen Nitroglycerin (NTG), Isosorbidmononitrat (ISMN) oder Isosorbiddinitrat (ISDN) enthält:
| | |
|---|---|
| NTG Retard, peroral: | 0,5-31,2 mg; |
| NTG transdermales Pflaster: | 0,2-0,8 mg/h; |
| ISDN Tablette oder Kapsel: | 0,3-135 mg; |
| ISDN Retard, peroral: | 0,2-160 mg; |
| ISDN transdermal: | 3-180 mg; |
| ISMN Tablette oder Kapsel: | 1-40 mg; |
| ISMN Retard, peroral: | 2-240 mg; |
| ISMN transdermal: | 40-300 mg. |

13. Verwendung eines als enzymatischer Stickstoffoxid(NO)-Donator wirkenden organischen Nitrats nach Anspruch 3, bei welcher die Zusammensetzung die nachfolgend genannten Tagesdosen Nitroglycerin (NTG), Isosorbidmononitrat (ISMN) oder Isosorbiddinitrat (ISDN) enthält:
| | |
|---|---|
| NTG Retard, peroral: | 0,5-5,2 mg; |
| ISDN Tablette oder Kapsel: | 0,3-3 mg; |
| ISDN Retard, peroral: | 0,2-2 mg; |
| ISMN Tablette oder Kapsel: | 1-20 mg; |
| ISMN Retard, peroral: | 2-30 mg; |
| ISMN transdermal: | 40-3.00 mg. |

14. Verwendung eines als enzymatischer Stickstoffoxid(NO)-Donator wirkenden organischen Nitrats nach einem der Ansprüche 4 bis 11, bei welcher das Medikament für die Kombinationstherapie-Behandlung bestimmt ist und das Medikament eine Zusammensetzung nach Anspruch 5 ist oder eine Kombination von Zusammensetzungen nach Anspruch 6 umfasst und die Zusammensetzung(en) die nachfolgend genannten Dosen von NO-Donator (a) bzw. von antidiabetischem Wirkstoff (b) enthält bzw. enthalten:
a) als enzymatischer NO-Donator:
| | |
|---|---|
| NTG Retard, peroral | 0,26-31,2 mg; |
| ISDN peroral | 0,1-135 mg |
| ISDN Retard, peroral | 0,2-160 mg; |
| ISDN transdermal | 3-180 mg; |
| ISMN peroral | 0,1-120 mg; |
| ISMN Retard peroral | 0,2-240 mg; |
b) und als antidiabetischer Wirkstoff:
| | |
|---|---|
| Glibenclamid, peroral | 0,75-14 mg; |
| Metformin, peroral | 50-3000 mg; |
| Glyburid, peroral | 0,1-100 mg; |
| Idazoxan, peroral | 20-600 mg; |
| Troglitazon, peroral | 20-600 mg; |
| Pioglitazon, peroral | 5-50 mg; |
| Rosiglitazon, peroral | 5-50 mg; |
| Insulin, i.v. | 4-500 NE/ml. |

15. Verwendung eines als enzymatischer Stickstoffoxid(NO)-Donator wirkenden organischen Nitrats nach einem der Ansprüche 4 bis 11, bei welcher das Medikament eine der nachfolgend aufgeführten Kombinationen von zwei oder mehr Wirkstoffen umfasst:
- enzymatischer NO-Donator, Sulfonylharnstoff- und Biguanid-Derivat; or
- enzymatischer NO-Donator, Sulfonylharnstoff- und Thiazolidindion-Derivat; oder
- enzymatischer NO-Donator, Sulfonylharnstoff und Insulin; oder
- enzymatischer NO-Donator, Biguanid-Derivat und Thiazolidindion-Derivat; oder
- enzymatischer NO-Donator, Biguanid-Derivat und Insulin; oder
- enzymatischer NO-Donator, Thiazolidindion-Derivat und/oder Insulin; oder
- enzymatischer NO-Donator, Sulfonylharnstoff, Biguanid-und Thiazolidindion-Derivat; oder
- enzymatischer NO-Donator, Sulfonylharnstoff, Biguanid, Thiazolidindion-Derivat und Insulin.

16. Ein insulinsensibilisierendes Medikament, umfassen eine zur Insulinsensibilisierung wirksame Dosis wenigstens eines als enzymatischer Stickstoffoxid(NO)-Donator wirkenden organischen Nitrats und wenigstens eines antidiabetischen Wirkstoffes, der aus der Gruppe ausgewählt ist, die aus Thiazolidindion, Biguanid-Derivaten, α-Glucosidase-Inhibitor, α2-adrenergem Antagonist, Sulfonamiden, vorzugsweise einem Sulfonylharnstoff, Troglitazon, Pioglitazon, Rosiglitazon, Meglitinid-Analoga, Acetohexamid, Carbutamid, Chlorpropamid, Glibenclamid, Glibornurid, Glibutamid, Gliclazid, Glipizid, Glimeperid, Gliquidon, Glisentid, Glisolamid, Glisoxepid, Glybuzol, Glyclopyramid, Glycyclamid, Glymidin (freie Säure and ihre Salze), Metahexamid, Tolazamid, Tolbutamid, Metformin, Phenformin, Buformin, Idazoxan, Acarbose, Miglitol und Voglibose besteht.

17. Medikament nach Anspruch 16, **dadurch gekennzeichnet, dass** das Medikament eine pharmazeutische Zusammensetzung ist, die sowohl den bzw. die NO-Donator(en) als auch den bzw. die antidiabetischen Wirkstoff(e) enthält und wahlweise pharmazeutisch akzeptable Träger und/oder Hilfsstoffe umfasst.

18. Medikament nach Anspruch 16, **dadurch gekennzeichnet, dass** das Medikament eine Kombination von wenigstens zwei getrennten pharmazeutischen Zusammensetzungen umfasst, wobei eine der wenigstens zwei Zusammensetzungen eine wirksame Dosis des wenigstens einen NO-Donators umfasst, wenigstens eine weitere Zusammensetzung des Medikaments eine wirksame Dosis des wenigstens einen antidiabetischen Wirkstoffs umfasst und die Zusammensetzung(en) wahlweise pharmazeutisch akzeptable Träger und/oder andere Hilfsstoffe umfasst bzw. umfassen.

19. Medikament nach einem der Ansprüche 16 bis 18, **dadurch gekennzeichnet, dass** der bzw. die NO-Donator(en) aus der Gruppe ausgewählt ist/sind, die aus Nitroglycerin (NTG), racemischem Isosorbidmononitrat (ISMN) und/oder dessen Stereoisomeren, racemischem Isosorbiddinitrat (ISDN) und/oder dessen Stereoisomeren, Erythrityltetranitrat, Pentaerythritoltetranitrat, Methylpropylpropandioldinitrat, Propatylnitrat, Trolnitrat, Tenitramin und Nicorandil besteht.

20. Medikament nach einem der Ansprüche 17 bis 19, **dadurch gekennzeichnet, dass** die pharmazeutischen Zusammensetzung(en) für die parenterale, vorzugsweise intravenöse, intramuskuläre, subkutane oder transdermale, oder für die orale, nasale, sublinguale oder bukkale Verabreichung formuliert ist/sind, wobei die oralen Formulierungen flüssige Zubereitungen, vorzugsweise Flüssigkeiten und Sprays, und feste Zubereitungen, vorzugsweise Tabletten, umfassen.

21. Medikament nach einem der Ansprüche 16 bis 20, **dadurch gekennzeichnet, dass** es des Weiteren Insulin enthält.

22. Medikament nach einem der Ansprüche 16 bis 21, **dadurch gekennzeichnet, dass** es eine der folgenden Kombinationen von zwei oder mehr Wirkstoffen enthält:
- enzymatischer NO-Donator, Sulfonylharnstoff- und Biguanid-Derivat; oder
- enzymatischer NO-Donator, Sulfonylharnstoff- und Thiazolidindion-Derivat; oder
- enzymatischer NO-Donator, Sulfonylharnstoff und Insulin; oder
- enzymatischer NO-Donator, Biguanid-Derivat und Thiazolidindion-Derivat; oder
- enzymatischer NO-Donator, Biguanid-Derivat und Insulin; oder
- enzymatischer NO-Donator, Thiazolidindion-Derivat und/oder Insulin; oder
- enzymatischer NO-Donator, Sulfonylharnstoff, Biguanid-und Thiazolidindion-Derivat; oder
- enzymatischer NO-Donator, Sulfonylharnstoff, Biguanid, Thiazolidindion-Derivat und Insulin.

## Revendications

1. Utilisation d'au moins un nitrate organique donneur d'oxyde nitrique (NO) enzymatique pour la fabrication d'un médicament insulinosensibilisant pour :
- le traitement du diabète sucré ;
- la prévention du diabète sucré ;
- le traitement du prédiabète et des affections prédiabétiques ;
- la prévention des affections prédiabétiques ;
- le traitement d'une maladie associée au diabète choisie parmi le groupe constitué : des perturbations de la mobilité gastrique et intestinale, en particulier, la gastroparésie ; des problèmes affectant le sphincter de Oddi ;
dans laquelle ledit médicament contient une dose efficace insulinosensibilisante dudit au moins un nitrate organique donneur de NO enzymatique.

2. Utilisation de l'au moins un donneur de NO enzymatique selon la revendication 1, **caractérisée en ce que** lesdites affections prédiabétiques sont choisies parmi le groupe du syndrome des ovaires polykystiques et du syndrome du diabète gestationnel.

3. Utilisation d'un nitrate organique donneur d'oxyde nitrique (NO) enzymatique selon la revendication 1, dans laquelle ledit médicament est une composition pharmaceutique contenant le(s)dit(s) donneur(s) de NO, et qui comprend facultativement des supports et/ou d'autres auxiliaires pharmaceutiquement acceptables, ladite composition étant de préférence formulée sous forme d'une composition pour l'administration orale liquide, orale solide, nasale ou sublinguale et davantage préféré sous forme de comprimés.

4. Utilisation d'un nitrate organique donneur d'oxyde nitrique (NO) enzymatique selon l'une quelconque des revendications 1 à 3, dans laquelle ledit médicament comprend en plus une dose efficace d'au moins un ingrédient antidiabétique actif, de préférence une dose efficace d'au moins un ingrédient antidiabétique actif oral.

5. Utilisation d'un nitrate organique donneur d'oxyde nitrique (NO) enzymatique selon la revendication 4, dans laquelle ledit médicament est une composition pharmaceutique contenant à la fois le(s) donneur(s) de NO et le(s)dit(s) ingrédient(s) antidiabétique(s) et comprend facultativement des supports et/ou d'autres auxiliaires pharmaceutiquement acceptables.

6. Utilisation d'un nitrate organique donneur d'oxyde nitrique (NO) enzymatique selon la revendication 4, dans laquelle ledit médicament comprend une combinaison d'au moins deux compositions pharmaceutiques séparées, et dans laquelle une desdites au moins deux compositions comprend une dose efficace dudit au moins un donneur de NO et au moins une autre composition de ladite combinaison comprend une dose efficace dudit au moins un ingrédient antidiabétique et ladite (lesdites) composition(s) comprend (comprennent) des supports et/ou d'autres auxiliaires pharmaceutiquement acceptables.

7. Utilisation d'un nitrate organique donneur d'oxyde nitrique (NO) enzymatique selon l'une quelconque des revendications 1 à 6, dans laquelle le(s)dit(s) de NO est (sont) choisi(s) parmi le groupe constitué de la nitroglycérine (NTG), du mononitrate d'isosorbide racémique (ISMN) et/ou de ses stéréoisomères, du dinitrate d'isosorbide racémique (ISDN) et/ou de ses stéréoisomères, du tétranitrate d'érythrityle, du tétranitrate de pentaérytritol, du dinitrate de méthylpropyl-propanediol, du nitrate de propatyle, du trolnitrate, de la tenitramine et/ou du nicorandile.

8. Utilisation d'un nitrate organique donneur d'oxyde nitrique (NO) enzymatique selon l'une quelconque des revendications 4 à 7, dans laquelle le(s)dit(s) ingrédient(s) antidiabétique (s) est (sont) choisi(s) parmi le groupe constitué de l'insuline, la thiazolidinedione, les dérivés de biguanides, un inhibiteur de l'α-glucosidase, un antagoniste α2-adrénergique, des sulfonamides, de préférence une sulfonylurée, la troglitazone, la pioglitazone, la rosiglitazone, les analogues du meglitinide, l'acétohexamide, le carbutamide, le chlorpropamide, le glibenclamide, le glibornuride, le glibutamide, le gliclazide, le glipizide, le glimépiride, la gliquidone, le glisentide, le glisolamide, le glisoxepide, le glybuzole, le glyclopyramide, le glycyclamide, la glymidine acide libre et ses sels, le métahexamide, le tolazamide, le tolbutamide, la metformine, la phenformine, la buformine, l'idazoxane, l'acarbose, le miglitol et/ou le voglibose.

9. Utilisation d'un nitrate organique donneur d'oxyde nitrique (NO) enzymatique selon l'une quelconque des revendications 4 à 8, dans laquelle le(s)dit(s) donneur(s) de NO est (sont) choisi(s) parmi le groupe constitué de la nitroglycérine, du mononitrate d'isosorbide racémique et/ou de ses stéréoisomères, du dinitrate d'isosorbide racémique et/ou de ses stéréoisomères, et dans laquelle ledit ingrédient antidiabétique est choisi parmi le groupe de l'insuline, de la troglitazone, de la pioglitazone, de la rosiglitazone, du glenclamide, de la metformine et de l'idazoxane.

10. Utilisation d'un nitrate organique donneur d'oxyde nitrique (NO) enzymatique selon l'une quelconque des revendications 3 à 9, dans laquelle ladite (lesdites) composition(s) est (sont) formulée(s) pour l'administration parentérale, de préférence intraveineuse, intramusculaire, sous-cutanée ou transdermique, ou pour l'administration orale, nasale, sublinguale ou buccale, dans laquelle lesdites formulations orales comprennent des préparations liquides, de préférence des liquides et des sprays, et des préparations solides, de préférence des comprimés.

11. Utilisation d'un nitrate organique donneur d'oxyde nitrique (NO) enzymatique selon la revendication 10, dans laquelle ladite (lesdites) compositions est (sont) formulée(s) sous forme d'une formulation à libération prolongée, de préférence sous forme d'un médicament à libération prolongée pour l'application orale ou un timbre transdermique.

12. Utilisation d'un nitrate organique donneur d'oxyde nitrique (NO) enzymatique selon la revendication 3, dans laquelle ladite composition contient les doses quotidiennes suivantes, ou les doses horaires, de nitroglycérine (NTG), de mononitrate d' isosorbide (ISMN) ou de dinitrate d' isosorbide (ISDN) :
| | |
|---|---|
| NTG libération prolongée, per os : | 0,5-31,2 mg ; |
| NTG timbre transdermique : | 0,2-0,8 mg/h ; |
| ISDN comprimé ou capsule : | 0,3-135 mg ; |
| ISDN libération prolongée, per os : | 0,2-160 mg ; |
| ISDN transdermique : | 3-180 mg ; |
| ISMN comprimé ou capsule : | 1-40 mg ; |
| ISMN libération prolongée, per os : | 2-240 mg ; |
| ISMN transdermique : | 40-300 mg. |

13. Utilisation d'un nitrate organique donneur d'oxyde nitrique (NO) enzymatique selon la revendication 3, dans laquelle ladite composition contient les doses quotidiennes suivantes de nitroglycérine (NTG), de mononitrate d'isosorbide (ISMN) ou de dinitrate d'isosorbide (ISDN) :
| | |
|---|---|
| NTG libération prolongée, per os : | 0,5-5,2 mg ; |
| ISDN comprimé ou capsule : | 0,3-3 mg ; |
| ISDN libération prolongée, per os : | 0,2-2 mg ; |
| ISMN comprimé ou capsule : | 1-20 mg ; |
| ISMN libération prolongée, per os : | 2-30 mg ; |
| ISMN transdermique : | 40-300 mg. |

14. Utilisation d'un nitrate organique donneur d'oxyde nitrique (NO) enzymatique selon l'une quelconque des revendications 4 à 11, dans laquelle ledit médicament est destiné au traitement thérapeutique combiné et ledit médicament est une composition selon la revendication 5 ou comprend une combinaison des combinaisons comme définies dans la revendication 6, et ladite (lesdites) composition(s) contient (contiennent) les doses suivantes de donneur de NO (a) et d'ingrédient antidiabétique (b), respectivement :
a) comme donneur de NO enzymatique :
| | |
|---|---|
| NTG retard, per os : | 0,26-31,2 mg ; |
| ISDN, per os : | 0,1-135 mg ; |
| ISDN retard, per os : | 0,2-160 mg ; |
| ISDN transdermique : | 3-180 mg ; |
| ISMN per os : | 0,1-120 mg ; |
| ISMN retard, per os : | 0,2-240 mg ; |
b) et comme ingrédient antidiabétique actif :
| | |
|---|---|
| glibenclamide, per os : | 0,75-3000 mg ; |
| metformine, per os : | 50-3000 mg ; |
| glyburide, per os : | 0,1-100 mg ; |
| idazoxane, per os : | 20-600 mg ; |
| troglitazone, per os : | 20-600 mg ; |
| pioglitazone, per os : | 5-50 mg ; |
| rosiglitazone, per os : | 5-50 mg ; |
| insuline, i.v. : | 4-500 NE/ml. |

15. Utilisation d'un nitrate organique donneur d'oxyde nitrique (NO) enzymatique selon l'une quelconque des revendications 4 à 11, dans laquelle ledit médicament comprend une des combinaisons suivantes de deux substances actives ou plus :
- donneur de NO enzymatique, sulfonylurée et dérivé de biguanide ; ou
- donneur de NO enzymatique, sulfonylurée et dérivé de thiazolidinedione ; ou
- donneur de NO enzymatique, sulfonylurée et insuline ; ou
- donneur de NO enzymatique, dérivé de biguanide et dérivé de thiazolidinedione ; ou
- donneur de NO enzymatique, dérivé de biguanide et insuline ; ou
- donneur de NO enzymatique, dérivé de thiazolidinedione et/ou insuline ; ou
- donneur de NO enzymatique, sulfonylurée, dérivé de biguanide et de thiazolidinedione ; ou
- donneur de NO enzymatique, sulfonylurée, dérivé de biguanide et de thiazolidinedione et insuline.

16. Médicament insulinosensibilisant comprenant une dose efficace insulinosensibilisante d'au moins un nitrate organique donneur d'oxyde nitrique (NO) enzymatique et au moins un ingrédient antidiabétique actif choisi parmi le groupe constitué de la thiazolidinedione, des dérivés de biguanides, d'un inhibiteur de l'α-glucosidase, d'un antagoniste α2-adrénergique, des sulfonamides, de préférence une sulfonylurée, la troglitazone, la pioglitazone, la rosiglitazone, les analogues de meglitinide, l'acétohexamide, le carbutamide, le chlorpropamide, le glibenclamide, le glibornuride, le glibutamide, le, gliclazide, le glipizide, le glimépiride, la gliquidone, le glisentide, le glisolamide, le glisoxepide, le glybuzole, le glyclopyramide, le glycyclamide, la glymidine acide libre et ses sels, le métahexamide, le tolazamide, le tolbutamide, la metformine, la phenformine, la buformine, l'idazoxane, l'acarbose, le miglitol,

17. Médicament selon la revendication 16, **caractérisé en ce que** ledit médicament est une composition pharmaceutique contenant à la fois le(s)dit(s) donneur (s) de NO et le(s)dit(s) ingrédient(s) antidiabétique(s), et facultativement comprend des supports et/ou d'autres auxiliaires pharmaceutiquement acceptables.

18. Médicament selon la revendication 16, **caractérisé en ce que** ledit médicament comprend une composition d'au moins deux compositions pharmaceutiques séparées, et dans lequel une desdites au moins deux compositions comprend une dose efficace dudit au moins un donneur de NO et au moins une autre composition dudit médicament comprend une dose efficace dudit au moins un ingrédient antidiabétique, et ladite (lesdites) composition(s) comprend (comprennent) facultativement des supports et/ou d'autres auxiliaires pharmaceutiquement acceptables.

19. Médicament selon l'une quelconque des revendications 16 à 18, **caractérisé en ce que** le(s)dit(s) donneur(s) de NO est (sont) choisi(s) parmi le groupe constitué de la nitroglycérine (NTG), du mononitrate d'isosorbide racémique (ISMN) et/ou de ses stéréoisomères, du dinitrate d'isosorbide racémique (ISDN) et/ou de ses stéréoisomères, du tétranitrate d'érythrityle, du tétranitrate de pentaérytritol, du dinitrate de méthylpropyl-propanediol, du nitrate de propatyle, de trolnitrate, de la tenitramine et/ou du nicorandile.

20. Médicament selon l'une quelconque des revendications 17 à 19, **caractérisé en ce que** ladite (lesdites) composition(s) pharmaceutique(s) est (sont) formulée(s) pour l'administration parentérale, de préférence intraveineuse, intramusculaire, sous-cutanée ou transdermique, ou pour l'administration orale, nasale, sublinguale ou buccale, dans laquelle lesdites formulations orales comprennent des préparations liquides, de préférence des liquides et des sprays, et des préparations solides, de préférence des comprimés.

21. Médicament selon l'une quelconque des revendications 16 à 20, **caractérisé en ce qu'**il contient de l'insuline.

22. Médicament selon l'une quelconque des revendications 16 à 21, **caractérisé en ce qu'**il comprend une des combinaisons suivantes de deux substances actives ou plus :
- donneur de NO enzymatique, sulfonylurée et dérivé de biguanide ; ou
- donneur de NO enzymatique, sulfonylurée et dérivé de thiazolidinedione ; ou
- donneur de NO enzymatique, sulfonylurée et insuline ; ou
- donneur de NO enzymatique, dérivé de biguanide et dérivé de thiazolidinedione ; ou
- donneur de NO enzymatique, dérivé de biguanide et insuline ; ou
- donneur de NO enzymatique, dérivé de thiazolidinedione et/ou insuline ; ou
- donneur de NO enzymatique, sulfonylurée, dérivé de biguanide et de thiazolidinedione ; ou
- donneur de NO enzymatique, sulfonylurée, dérivé de biguanide et de thiazolidinedione et insuline.
